# NEW EUROPEAN PATENT SPECIFICATION

(11) **EP 1 251 744 B2**
(45) Date of publication and mention of the opposition decision: **26.08.2015**
(45) Mention of the grant of the patent: 03.10.2007
(21) Application number: 01903376.0
(22) Date of filing: 26.01.2001
(51) Int. Cl.: A23D 9/00, A23L 1/03, C12N 1/00, C12P 7/64

(54) **ENHANCED PRODUCTION OF LIPIDS CONTAINING POLYENOIC FATTY ACIDS BY HIGH DENSITY CULTURES OF EUKARYOTIC MICROBES IN FERMENTORS**
VERSTAERKTE PRODUKTION VON LIPIDEN ENTHALTEND MEHRFACHUNGESAETTIGTE FETTSAEUREN DURCH HOCHDICHTE KULTUREN VON EUKARIOTISCHEN MIKROBEN IN GAERVORRICHTUNGEN
PRODUCTION AMELIOREE DE LIPIDES CONTENANT DES ACIDES GRAS POLYENES AU MOYEN DE CULTURES A GRANDE DENSITE DE MICROBES EUCARYOTES DANS DES FERMENTEURS

(30) Priority: 28.01.2000 US 178588 P
(43) Date of publication of application: 30.10.2002
(60) Divisional application: 06076087.3
(73) Proprietor: DSM IP Assets B.V., 6411 TE Heerlen (NL)
(72) Inventor: RUECKER, Craig, M., San Diego, CA 92129 (US); DIMASI, Don, San Diego, CA 92129 (US); HANSEN, Jon, M., Chula Vista, CA 91910 (US); MIRRASOUL, Peter, J., San Diego, CA 92107 (US); BAILEY, Richard, B., Del Mar, CA 92014 (US); WEEDER, George, T., III, Ramona, CA 92126 (US); KANEKO, Tatsuo, San Diego, CA 92126 (US); BARCLAY, William, R., Boulder, CO 80303 (US)
(74) Representative: D Young & Co LLP
(86) International application number: PCT/US2001/002715
(87) International publication number: WO 2001/054510

(56) References cited:
- EP-A1- 0 823 475
- EP-A2- 1 024 199
- WO-A-98/03671
- WO-A1-92/13086
- WO-A1-94//08467
- WO-A1-98//55625
- WO-A2-98/37179
- DE-A1- 19 838 011
- US-A- 5 130 242
- US-A- 5 244 921
- YAZAWA K ET AL: "PRODUCTION OF EICOSAPENTAENOIC ACID FROM MARINE BACTERIA" INDUSTRIAL APPLICATIONS OF SINGLE CELL OILS, XX, XX, 1992, pages 29-51, XP001000790
- WALLIS J G ET AL: "Polyunsaturated fatty acid synthesis: what will they think of next?" TIBS TRENDS IN BIOCHEMICAL SCIENCES, ELSEVIER PUBLICATION, CAMBRIDGE, EN, vol. 27, no. 9, 1 September 2002 (2002-09-01), pages 467-473, XP004378766 ISSN: 0968-0004
- METZ J G ET AL: "Production of polyunsaturated fatty acids by polyketide synthases in both prokaryotes and eukaryotes" SCIENCE, AMERICAN ASSOCIATION FOR THE ADVANCEMENT OF SCIENCE,, US, vol. 293, 13 July 2001 (2001-07-13), pages 290-293, XP002956819 ISSN: 0036-8075
- SOGIN ET AL. STRAMENOPILES, [Online] 1995, pages 1 - 3, XP002939654 Retrieved from the Internet: <URL:http://ag.arizona.edu/tree/eukaryotes/ stramenopiles/stramenopiles.html> [retrieved on 2001-03-07]
- BEACH D. H. ET AL: 'Environmental influences on the docosahexaenoate content of the triacyglycerols and phosphatidylcholine of a heterotrophic. Marine dinoflagellate. Crypthecodinium cohnii' BIOCHIMICA ET BIOPHISICA ACTA no. 316, 1973, pages 56 - 65

## Description

### FIELD OF THE INVENTION

The present invention is directed to a novel process for growing microorganisms and recovering microbial lipids. In particular, the present invention is directed to producing microbial polyunsaturated lipids.

### BACKGROUND OF THE INVENTION

Production of polyenoic fatty acids (fatty acids containing 2 or more unsaturated carbon-carbon bonds) in eukaryotic microorganisms has been generally believed to require the presence of molecular oxygen (*i.e.,* aerobic conditions). This is because it is believed that the cis double bond formed in the fatty acids of all non-parasitic eukaryotic microorganisms involves a direct oxygen-dependent desaturation reaction (oxidative microbial desaturase systems). Other eukaryotic microbial lipids that are known to require molecular oxygen include fungal and plant sterols, oxycarotenoids (*i.e.*, xanthophylls), ubiquinones, and compounds made from any of these lipids (*i.e.*, secondary metabolites).

Certain eukaryotic microbes (such as algae; fungi, including yeast; and protists) have been demonstrated to be good producers ofpolyenoic fatty acids in fermentors. However, very high density cultivation (greater than about 100 g/L microbial biomass, especially at commercial scale) can lead to decreased polyenoic fatty acid contents and hence decreased polyenoic fatty acid productivity. This may be due in part to several factors including the difficulty of maintaining high dissolved oxygen levels due to the high oxygen demand developed by the high concentration of microbes in the fermentation broth. Methods to maintain higher dissolved oxygen level include increasing the aeration rate and/or using pure oxygen instead of air for aeration and/or increasing the agitation rate in the fermentor. These solutions generally increase the cost of lipid production and capital cost of fermentation equipment, and can cause additional problems. For example, increased aeration can easily lead to severe foaming problems in the fermentor at high cell densities and increased mixing can lead to microbial cell breakage due to increased shear forces in the fermentation broth (this causes the lipids to be released in the fermentation broth where they can become oxidized and/or degraded by enzymes). Microbial cell breakage is an increased problem in cells that have undergone nitrogen limitation or depletion to induce lipid formation, resulting in weaker cell walls.

As a result, when lipid-producing eukaryotic microbes are grown at very high cell concentrations, their lipids generally contain only very small amounts of polyenoic fatty acids. For example, the yeast *Lipomyces starkeyi* has been grown to a density of 153 g/L with resulting lipid concentration of 83 g/L in 140 hours using alcohol as a carbon source. Yet the polyenoic fatty acid content of the yeast at concentration greater than 100 g/L averaged only 4.2% of total fatty acids (dropping from a high of 11.5% of total fatty acid at a cell density of 20-30 g/L). Yamauchi et al.,J. Ferment. Technol., 1983, 61, 275-280. This results in a polyenoic fatty acid concentration of only about 3.5 g/L and an average polyenoic fatty acid productivity of only about 0.025 g/L/hr. Additionally, the only polyenoic fatty acid reported in the yeast lipids was C18:2.

Another yeast, *Rhodotorula glutinus,* has been demonstrated to have an average lipid productivity of about 0.49 g/L/hr, but also a low overall polyenoic fatty acid content in its lipids (15.8% of total fatty acids, 14.7% C18:2 and 1.2% C18:3) resulting in a polyenoic fatty acid productivity in fed-batch culture of only about 0.047 g/L/hr and 0.077 g/L/hr in continuous culture.

One of the present inventors has previously demonstrated that certain marine microalgae in the order Thraustochytriales can be excellent producers of polyenoic fatty acids in fermentors, especially when grown at low salinity levels and especially at very low chloride levels. Others have described Thraustochytrids that exhibit an average polyenoic fatty acid (DHA, C22:6n-3; and DPA, C22:Sn-6) productivity of about 0.158 g/L/hr, when grown to cell density of 59 g/L in 120 hours. However, this productivity was only achieved at a salinity of about 50% seawater, a concentration that would cause serious corrosion in conventional stainless steel fermentors.

Costs of producing microbial lipids containing polyenoic fatty acids, and especially the highly unsaturated fatty acids, such as C18:4n-3, C20:4n-6, C20:5n3, C22:5n-3, C22:5n-6 and C22:6n-3, have remained high in part due to the limited densities to which the high polyenoic fatty acid containing eukaryotic microbes have been grown and the limited oxygen availability both at these high cell concentrations and the higher temperatures needed to achieve high productivity.

Therefore, there is a need for a process for growing microorganisms at high concentration which still facilitates increased production of lipids containing polyenoic fatty acids.

WO-A-92/13086 discloses a method for the production of an arachidonic acid-containing oil substantially free of eicosapentaneoic acid, comprising cultivating a species of *Pythium* which produces an arachidonic oil substantially free of eicosapentaneoic acid under suitable oil-producing-cultivating conditions; harvesting said *Pythium;* extracting said oil from said harvested *Pythium,* and recovering said oil.

### SUMMARY OF THE INVENTION

According to the present invention there is provided a process for producing microbial lipids comprising:
(a) conducting a fermentation of a medium comprising microorganisms, a carbon source and a limiting nutrient source and providing conditions sufficient to maintain a dissolved oxygen level of at least about 4% of saturation in said fermentation medium to increase the biomass density;
(b) subsequently providing conditions sufficient to maintain a dissolved oxygen level of about 1% or less of saturation in said fermentation medium and providing conditions sufficient to allow said microorganisms to produce said lipids; and
(c) recovering said microbial lipids,
wherein at least about 15% of said microbial lipids are polyunsaturated lipids; and
wherein a biomass density of at least 100 g/L is achieved during the fermentation wherein said microorganisims are order Thraustochytriales.

We describe a process for enriching the polyenoic fatty acid content of a microorganism comprising fermenting said microorganism in a growth medium having a level of dissolved oxygen of less than 1% of saturation.

We describe a heterotrophic process for producing products and microorganisms comprising culturing said microorganisms in a growth medium having a level of dissolved oxygen at less than about 1 % of saturation wherein said microorganisms contain polyketide synthase genes.

Also disclosed is a process for growing eukaryotic microorganisms that are capable of producing at least about 20% of their biomass as lipids and a method for producing the lipids. Preferably the lipids contain one or more polyenoic fatty acids. Embodiments of the process comprise adding to a fermentation medium comprising eukaryotic microorganisms a carbon source, preferably a non-alcohol carbon source, and a limiting nutrient source. Preferably, the carbon source and the limiting nutrient source are added at a rate sufficient to increase the biomass density of the fermentation medium to at least about 100 g/L.

In one embodiment of the present invention, the fermentation condition comprises a biomass density increasing stage and a lipid production stage, wherein the biomass density increasing stage comprises adding the carbon source and the limiting nutrient nitrogen source, and the lipid production stage comprises adding the carbon source without adding the limiting nutrient source to create conditions which induce lipid production.

Particularly useful microorganisms of the present invention are microorganisms of the order Thraustochytriales that are capable of producing lipids at a fermentation medium oxygen level of about less than 3% of saturation.

In still another embodiment of the present invention, microorganisms are grown in a fed-batch process.

Also disclosed is a process for producing eukaryotic microbial lipids comprising:
(a) growing eukaryotic microorganisms in a fermentation medium to increase the biomass density of said fermentation medium to at least about 100 g/L;
(b) providing fermentation conditions sufficient to allow said microorganisms to produce said lipids; and
(c) recovering said lipids,
   wherein greater than about 15% of said lipids are polyunsaturated lipids.

Another aspect of the present invention provides a lipid recovery process that comprises:
(d) removing water from said fermentation medium to provide dry microorganisms; and
(e) isolating said lipids from said dry microorganisms.

Preferably, the water removal step comprises contacting the fermentation medium directly on a drumdryer without prior centrifugation. Another embodiment of the present invention provides a lipid recovery process that comprises:
(d) treating the fermentation broth to permeabilize, lyse or rupture the microbial cells; and
(e) recovering the lipids from the fermentation broth by gravity separation, and preferably centrifugation, with or without the aid of a water-soluble solvent to aid in breaking the lipid/water emulsion.

Preferably, the microbial cells are treated in step (c) in a fermentor or a similar vessel.

### BRIEF DESCRIPTION OF THE DRAWINGS

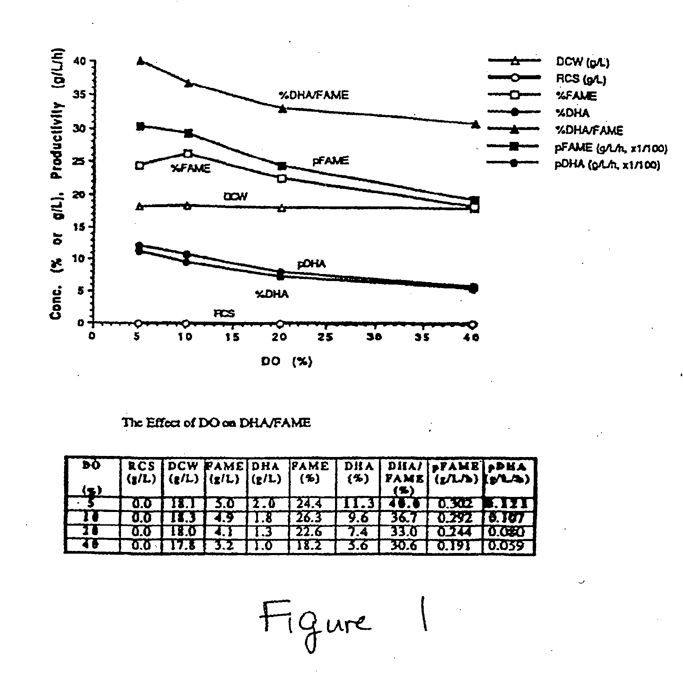

Figure 1 is a table and a plot of various lipid production parameters of a microorganism versus the amount of dissolved oxygen in a fermentation medium.

### DETAILED DESCRIPTION OF THE INVENTION

The present invention relates to a process for growing microorganisms of the order Thraustochytriales. Moreover, the process of the present invention can be used to produce a variety of lipid compounds, in particular unsaturated lipids, preferably polyunsaturated lipids *(i.e.,* lipids containing at least 2 unsaturated carbon-carbon bonds, *e.g.* double bonds), and more preferably highly unsaturated lipids (*i.e*., lipids containing 4 or more unsaturated carbon-carbon bonds) such as omega-3 and/or omega-6 polyunsaturated fatty acids, including docosahexaenoic acid *(i.e.,* DHA); and other naturally occurring unsaturated, polyunsaturated and highly unsaturated compounds. As used herein, the term "lipid" includes phospholipids; free fatty acids; esters of fatty acids; triacylglycerols; sterols and sterol esters; carotenoids; xanthophylls (*e.g.*, oxycarotenoids); hydrocarbons; isoprenoid-derived compounds and other lipids known to one of ordinary skill in the art.

Specifically, processes of the present invention are useful in growing microorganisms of the order Thraustochytriales that produce polyenoic fatty acid(s), and for producing microbial polyenoic fatty acid(s).

While processes of the present invention can be used to grow a wide variety of microorganisms of the order Thraustochytriales and to obtain polyunsaturated lipid containing compounds produced by the same, for the sake of brevity, convenience and illustration, this detailed description of the invention will discuss processes for growing microorganisms of the order Thraustochytriales which are capable of producing lipids comprising omega-3 and/or omega-6 polyunsaturated fatty acids, in particular microorganisms of the order Thraustochytriales that are capable of producing DHA (or closely related compounds such as DPA, EPA or ARA). Preferably the present invention relates to the use of Thraustochytriales of the genus *Thraustochytrium* and *Schizochytrium*, including Thraustochytriales which are disclosed in commonly assigned U.S. Patent Nos. 5,340,594 and 5,340,742, both issued to Barclay. It should be noted that many experts agree that *Ulkenia* is not a separate genus, but is in fact part of the genus *Schizochytrium.* As used herein, the genus *Schizochytrium* will include *Ulkenia.*

Preferred microorganisms are those that produce the compounds of interest via polyketide synthase systems. Such microorganisms include microorganisms having an endogenous polyketide synthase system and microorganisms into which a polyketide synthase system has been genetically engineered. Polyketides are structurally diverse natural products that have a wide range of biological activities, including antibiotic and pharmacological properties. Biosynthesis of the carbon chain backbone of polyketides is catalyzed by polyketide synthases. Like the structurally and mechanistically related fatty acid synthases, polyketide synthases catalyze the repeated decarboxylative condensations between acyl thioesters that extend the carbon chain two carbons at a time. However, unlike fatty acid synthases, polyketide synthases can generate great structural variability in the end product. Individual polyketide synthase systems can do this by using starting units other than acetate, by utilizing methyl- or ethyl-malonate as the extending unit and by varying the reductive cycle of ketoreduction, dehydration and enoyl reduction on the beta-keto group formed after each condensation. Of particular interest here is that the carbon-carbon double bonds that are introduced by the dehydration step can be retained in the end product Further, although these double bonds are initially in the *trans* configuration, they can be converted to the *cis* configuration found in DHA (and other polyenoic fatty acids of interest) by enzymatic isomerization. Both the dehydrase and isomerization reactions can occur in the absence of molecular oxygen.

We describe a heterotrophic process for producing products and microorganisms. The process may comprise culturing the microorganisms in a growth medium wherein the microorganisms contain a polyketide synthase system. The level of dissolved oxygen is maintained at less than about 1 percent.

Assuming a relatively constant production rate of lipids by an algae, it is readily apparent that the higher biomass density will lead to a higher total amount of lipids being produced per volume. Current conventional fermentation processes for growing algae yield a biomass density of from about 50 to about 80 g/L or less. The present inventors have found that by using processes of the present invention, a significantly higher biomass density than currently known biomass density can be achieved. Preferably, processes of the present invention produces biomass density of at least about 100 g/L, more preferably at least about 130 g/L, still more preferably at least about 150 g/L, yet still more preferably at least about 170 g/L, and most preferably greater than 200 g/L. Thus, with such a high biomass density, even if the lipids production rate of algae is decreased slightly, the overall lipids production rate per volume is significantly higher than currently known processes.

Processes for growing microorganisms of the order Thraustochytriales include adding a source of carbon and a source of a limiting nutrient to a fermentation medium comprising the microorganisms at a rate sufficient to increase the biomass density of the fermentation medium to those described above. As used herein, the term "limiting nutrient source" refers to a source of a nutrient (including the nutrient itself) essential for the growth of a microorganism in that, when the limiting nutrient is depleted from the growth medium, its absence substantially limits the microorganism from growing or replicating further. However, since the other nutrients are still in abundance, the organism can continue to make and accumulate intracellular and/or extracellular products. By choosing s specific limiting nutrient, one can control the type of products that are accumulated. Therefore, providing a limiting nutrient source at a certain rate allows one to control both the rate of growth of the microorganism and the production or accumulation of desired products (*e.g.*, lipids). This fermentation process, where one or more substrates *(e.g.*, a carbon source and a limiting nutrient source) are added in increments, is generally referred to as a fed-batch fermentation process. It has been found that when the substrate is added to a batch fermentation process the large amount of carbon source present (*e.g.*, about 200 g/L or more per 60 g/L of biomass density) had a detrimental effect on the microorganisms. Without being bound by any theory, it is believed that such a high amount of carbon source causes detrimental effects, including osmotic stress, for microorganisms and inhibits initial productivity of microorganisms. Processes of the present invention avoid this undesired detrimental effect while providing a sufficient amount of the substrate to achieve the above-described biomass density of the microorganisms.

Processes for growing microorganisms can include a biomass density increasing stage. In the biomass density increasing stage, the primary objective of the fermentation process is to increase the biomass density in the fermentation medium to obtain the biomass density described above. The rate of carbon source addition is typically maintained at a particular level or range that does not cause a significant detrimental effect on productivity of microorganisms, or the viability of the microorganisms resulting from insufficient capabilities of the fermentation equipment to remove heat from and transfer gases to and from the liquid broth An appropriate range of the amount of carbon source needed for a particular microorganism during a fermentation process is well known to one of ordinary skill in the art Preferably, a carbon source of the present invention is a non-alcohol carbon source, *i.e.*, a carbon source that does not contain alcohol. As used herein, an "alcohol" refers to a compound having 4 or less carbon atoms with one hydroxy group, *e.g.*, methanol, ethanol and isopropanolbut for the purpose of this invention does not include hydroxyl organic acids such as lactic acid and similar compounds. More preferably, a carbon source of the present invention is a carbohydrate, including, but not limited to, fructose, glucose, sucrose, molasses, and starch. Other suitable simple and complex carbon sources and nitrogen sources are disclosed in the above-referenced patents. Typically, however, a carbohydrate, preferably com syrup, is used as the primary carbon source. Fatty acids, in the form of hydroxy fatty acids, triglycerides, and di- and monoglycerides can also serve as the carbon source.

Particularly preferred nitrogen sources are urea, nitrate, nitrite, soy protein, amino acids, protein com steep liquor, yeast extract, animal by-products, inorganic ammonium salt, more preferably ammonium salts of sulfate, hydroxide, and most preferably ammonium hydroxide. Other limiting nutrient sources include carbon sources (as defined above), phosphate sources, vitamin sources (such as vitamin B₁₂ sources, pantothenate sources, thiamine sources), and trace metal sources (such as zinc sources, copper sources, cobalt sources, nickel sources, iron sources, manganese sources, molybdenum sources), and major metal sources (such as magnesium sources, calcium sources, , sodium sources, potassium sources, and silica sources, etc.). Trace metal sources and major metal sources can include sulfate and chloride salts of these metals (for example but not limited to MgSO₄•7H₂O; MnCl₂•4H₂O; ZnSO₄•7H₂O; CoCl₂•6H₂O;
Na₂MoO₄•2H₂O; CuSO₄•5H₂O; NiSO₄•6H₂O; FeSO₄•7H₂O; CaCl₂; K₂SO₄; KCI; and Na₂SO₄).

When ammonium is used as a nitrogen source, the fermentation medium becomes acidic if it is not controlled by base addition or buffers. When ammonium hydroxide is used as the primary nitrogen source, it can also be used to provide a pH control. The microorganisms of the order Thraustochytriales, in particular Thraustochytriales of the genus *Thraustochytrium* and *Schizochytrium,* will grow over a wide pH range, *e.g.,* from about pH 5 to about pH 11. A proper pH range for fermentation of a particular microorganism is within the knowledge of one skilled in the art.

Processes for growing microorganisms can also include a production stage. In this stage, the primary use of the substrate by the microorganisms is not increasing the biomass density but rather using the substrate to produce lipids. It should be appreciated that lipids are also produced by the microorganisms during the biomass density increasing stage; however, as stated above, the primary goal in the biomass density increasing stage is to increase the biomass density. Typically, during the production stage the addition of the limiting nutrient substrate is reduced or preferably stopped.

It was previously generally believed that the presence of dissolved oxygen in the fermentation medium is crucial in the production of polyunsaturated compounds, including omega-3 and/or omega-6 polyunsaturated fatty acids, by eukaryotic microorganisms. Thus, a relatively large amount of dissolved oxygen in the fermentation medium was generally believed to be preferred. Surprisingly and unexpectedly, however, the present inventors have found that the production rate of lipids is increased dramatically when the dissolved oxygen level during the production stage is reduced. Thus, while the dissolved oxygen level in the fermentation medium during the biomass density increasing stage is at least about 4% of saturation, and preferably at least about 8% of saturation, during the production stage the dissolved oxygen in the fermentation medium is reduced to about 1% of saturation or less, and more preferably about 0% of saturation. At the beginning of the fermentation the DO can be at or near saturation and as the microbes grow it is allowed to drift down to these low DO setpoints. In the present invention, the amount of dissolved oxygen level in the fermentation medium varies during the fermentation process. For example, for a fermentation process with total fermentation time of from about 90 hours to about 100 hours, the dissolved oxygen level in the fermentation medium is maintained at about 8% during the first 24 hours, about 4% from about 24^{th} hour to about 40^{th} hour, and about 0.5% or less from about 40^{th} hour to the end of the fermentation process.

The amount of dissolved oxygen present in the fermentation medium can be controlled by controlling the amount of oxygen in the head-space of the fermentor, or preferably by controlling the speed at which the fermentation medium is agitated (or stirred). For example, a high agitation (or stirring) rate results in a relatively higher amount of dissolved oxygen in the fermentation medium than a low agitation rate. For example, in a fermentor of about 14,000 gallon capacity the agitation rate is set at from about 50 rpm to about 70 rpm during the first 12 hours, from about 55 rpm to about 80 rpm during about 12^{th} hour to about 18^{th} hour and from about 70 rpm to about 90 rpm from about 18^{th} hour to the end of the fermentation process to achieve the dissolved oxygen level discussed above for a total fermentation process time of from about 90 hours to about 100 hours. A particular range of agitation speeds needed to achieve a particular amount of dissolved oxygen in the fermentation medium can be readily determined by one of ordinary skill in the art.

A preferred temperature for processes of the present invention is at least about 20°C, more preferably at least about 25°C, and most preferably at least about 30°C. It should be appreciated that cold water can retain a higher amount of dissolved oxygen than warm water. Thus, a higher fermentation medium temperature has the additional benefit of reducing the amount of dissolved oxygen, which is particularly desired as described above.

Certain microorganisms may require a certain amount of saline minerals in the fermentation medium. These saline minerals, especially chloride ions, can cause corrosion of the fermentor and other downstream processing equipment. To prevent or reduce these undesired effects due to a relatively large amount of chloride ions present in the fermentation medium, processes of the present invention can also include using non-chloride containing sodium salts, preferably sodium sulfate, in the fermentation medium as a source of sodium. More particularly, a significant portion of the sodium requirements of the fermentation is supplied as non-chloride containing sodium salts. For example, less than about 75% of the sodium in the fermentation medium is supplied as sodium chloride; more preferably less than about 50% and more preferably less than about 25%. The microorganisms of the present invention can be grown at chloride concentrations of less than about 3 g/L, more preferably less than about 500 mg/L, more preferably less than about 250 mg/L and more preferably between about 60 mg/L and about 120 mg/L.

Non-chloride containing sodium salts can include soda ash (a mixture of sodium carbonate and sodium oxide), sodium carbonate, sodium bicarbonate, sodium sulfate and mixtures thereof, and preferably include sodium sulfate. Soda ash, sodium carbonate and sodium bicarbonate tend to increase the pH of the fermentation medium, thus requiring control steps to maintain the proper pH of the medium. The concentration of sodium sulfate is effective to meet the salinity requirements of the microorganisms, preferably the sodium concentration is (expressed as g/L of Na) at least about 1 g/L, more preferably in the range of from about I g/L to about 50 g/L and more preferably in the range of from about 2 g/L to about 25 g/L.

Various fermentation parameters for inoculating, growing and recovering microorganisms are discussed in detail in U.S. Patent No. 5,130,242. Any currently known isolation methods can be used to isolate microorganisms from the fermentation medium, including centrifugation, filtration, ultrafiltration, decantation, and solvent evaporation. It has been found by the present inventors that because of such a high biomass density resulting from processes of the present invention, when a centrifuge is used to recover the microorganisms it is preferred to dilute the fermentation medium by adding water, which reduces the biomass density, thereby allowing more effective separation of microorganisms from the fermetnation medium.

The very high biomass densities achieved in the present invention also facilitate "solventless" processes for recovery of microbial lipids. Preferred processes for lysing the cells in the fermentor are d3escribed in U.S.. Provisional Patent Application Serial No. 60/177,125 entitled "Solventless Extraction Process" filed January 19, 2000; U.S. Patent Application Serial No. 09/766,500 entitled "Solventless Extraction Process" filed January 19, 2001 (now U.S. Patent No. 6,750,048), and PCT Patent Publication No. WO-A-0153512 entitled "Solventless Extraction Process" filed January 19, 2001. Preferred processes for recovering the lipids once the cells are permeabilized, broken or lysed in the fermentor (which enables the lipid emulsion to be broken, and the lipid-rich fraction to be recovered) include the deoiling process outlined in WO 96/05278 which is incorporated herein by reference in its entirety. In this process a water soluble compound, e.g., alcohol or acetone, is added to the oil/water emulsion to break the emulsion and the resulting mixture is separated by gravity separation, e.g., centrifugation. This process can also be modified to use other agents (water and/or lipid soluble) to break the emulsion.

Alternatively, the microorganisms are recovered in a dry form from the fermentation medium by evaporating water from the fermentation medium, *for example,* by contacting the fermentation medium directly (*i.e.*, without pre-concentration, for example, by centrifugation) with a dryer such as a drum-dryer apparatus, *i.e.*, a direct drum-dryer recovery process. When using the direct drum-dryer recovery process to isolate microorganisms, typically a steam-heated drum-dryer is employed. In addition when using the direct drum-dryer recovery process, the biomass density of the fermentation medium is preferably at least about 130 g/L, more preferably at least about 150 g/L, and most preferably at least about 180 g/L. This high biomass density is generally desired for the direct drum-dryer recovery process because-at a lower biomass density, the fermentation medium comprises a sufficient amount of water to cool the drum significantly, thus resulting in incomplete drying of microorganisms. Other methods of drying cells, including spray drying, are well known to one of ordinary skill in the art.

Processes of the present invention provide an average lipid production rate of at least about 0.5 g/L/hr, preferably at least about 0.7 g/L/hr, more preferably at least about 0.9 g/L/hr, and most preferably at least about 1.0 g/L/hr. Moreover, lipids produced by processes of the present invention contain polyunsaturated lipids in the amount greater than about 15%, preferably greater than about 20%, more preferably greater than about 25%, still more preferably greater than about 30%, and most preferably greater than about 35%. Lipids can be recovered from either dried microorganisms or from the microorganisms in the fermentation medium. Generally, at least about 20% of the lipids produced by the microorganisms in the processes of the present invention are omega-3 and/or omega-6 polyunsaturated fatty acids, preferably at least about 30% of the lipids are omega-3 and/or omega-6 polyunsaturated fatty acids, more preferably at least about 40% of the lipids are omega-3 and/or omega-6 polyunsaturated fatty acids, and most preferably at least about 50% of the lipids are omega-3 and/or omega-6 polyunsaturated fatty acids. Alternatively, processes of the present invention provides an average omega-3 fatty acid (e.g., DHA) production rate of at least about 0.2 g of omega-3 fatty acid (e.g., DHA)/L/hr, preferably at least about 0.3 g of omega-3 fatty acid (e.g., DHA)/L/hr, more preferably at least about 0.4 g of omega-3 fatty acid (e.g., DHA)/L/hr, and most preferably at least about 0.5 g of omega-3 fatty acid (e.g., DHA)/L/hr. Alternatively, processes of the present invention provide an average omega-6 fatty acid (e.g., DPAn-6) production rate of at least about 0.07 g of omega-6 fatty acid (e.g., DPAn-6)/L/hr, preferably at least about 0.1 g of omega-6 fatty acid (e.g., DPAn-6)/L/hr, more preferably at least about 0.13 g of omega-6 fatty acid (e.g., DPAn-6)/L/hr, and most preferably at least about 0.17 g of omega-6 fatty acid (e.g., DPAn-6)/L/hr. Still alternatively, at least about 25% of the lipid is DHA (based on total fatty acid methyl ester), preferably at least about 30%, more preferably at least about 35%, and most preferably at least about 40%.

Microorganisms, lipids extracted therefrom, the biomass remaining after lipid extraction or combinations thereof can be used directly as a food ingredient, such as an ingredient in beverages, sauces, dairy based foods (such as milk, yogurt, cheese and ice-cream) and baked goods; nutritional supplement (in capsule or tablet forms); feed or feed supplement for any animal whose meat or products are consumed by humans; food supplement, including baby food and infant formula; and pharmaceuticals (in direct or adjunct therapy application). The term "animal" means any organism belonging to the kingdom Animalia and includes, without limitation, any animal from which poultry meat, seafood, beef, pork or lamb is derived. Seafood is derived from, without limitation, fish, shrimp and shellfish. The term "products" includes any product other than meat derived from such animals, including, without limitation, eggs, milk or other products. When fed to such animals, polyunsaturated lipids can be incorporated into the -flesh, milk, eggs or other products of such animals to increase their content of these lipids.

Additional aims, advantages, and novel features of this invention are illustrated to those skilled in the art upon examination of the following examples thereof, which are not intended to be limiting.

### EXAMPLES

The strain of *Schizochytrium* used in these examples produces two primary polyenoic acids, DHAn-3 and DPAn-6 in the ratio of generally about 3:1, and small amounts of other polyenoic acids, such as EPA and C20:3, under a wide variety of fermentation conditions. Thus, while the following examples only list the amount of DHA, one can readily calculate the amount of DPA(n-6) produced by using the above-disclosed ratio.

### Reference Example 1

This example illustrates the effect of oxygen content in a fermentation medium on lipid productivity.

Fermentation results of *Schizochytrium* ATCC No. 20888 at various levels of dissolved oxygen content were measured The results are shown in Figure 1, where RCS is residual concentration of sugar, and DCW is dry-cell weight.

### Reference Example 2

This example also illustrates the effect of low oxygen content in the fermentation medium on DHA content (% dry weight) of the final biomass product.

A "scale-down" type experiment was conducted in 250 mL Erlenmeyer flasks to mimic the effect of low oxygen content on the DHA content in *Schizochytrium* sp. cells cultured in large-scale fermentors. *Schizochytrium* sp (ATCC 20888) was cultured in O4-4 medium. This culture media consisted of the following on a per liter basis dissolved in deionized water. Na₂SO₄ 12.61g; MgSO₄•7H₂O 1.2g; KCl 0.25 g; CaCl₂ 0.05 g; monosodium glutamate 7.0 g; glucose 10 g; KH₂PO₄ 0.5 g; NaHCO₃ 0.1 g; yeast extract 0.1 g; vitamin mix 1.0 mL; PII metals 1.00 mL. PII metal mix contains (per liter): 6.0 g Na₂EDTA, 0.29 g FeCl₃•6H₂O, 6.84 g H₃BO₃, 0.86 g MnCl₂•4H₂O, 0.06 g ZnCl₂, 0.026 g CoCl₂•6H₂O, 0.052 g NiSO₄•H₂O, 0.002 CuSO₄•H₂O and 0.005 g Na₂MoO₄•2H₂O. Vitamin mix contains (per liter): 100 mg thiamine, 0.5 mg biotin and 0.5 mg cyanocobalamin. The pH of the culture media was adjusted to 7.0 and it was then filter sterilized.

The idea behind this scale-down experiment was to culture the cells in shake flasks with different volumes of culture media in the flasks - almost full flasks (e.g. 200 mL in a 250 mL shake flask) would not mix well on a shake table and therefore as the cells grew, low dissolved oxygen conditions would be generated. Therefore 4 treatments were established in the experiment, each conducted in duplicate: (1) 250 mL flasks filled with 50 mL culture medium; (2) 250 mL flasks filled with 100 mL culture medium; (3) 250 mL flasks filled with 150 mL culture medium; and (4) 250 mL shake flasks filled with 200 mL culture medium. Each of the eight flasks was inoculated with cells from a 48 hour old culture of *Schizochytrium* cultured in O4-4 medium under the conditions in treatment 1, and at 28°C and 220 rpm on a shaker table. All eight flasks for the experiment were placed on a shaker table (220 rpm) in a incubator (28°C) and cultured for 48 hours in the dark. At the end of the experiment, dissolved oxygen (DO) levels in each flask were measured with a YSI dissolved oxygen meter, (YSI, Inc.), pH of the culture medium was also determined, and the dry weight of cells and their fatty acid content was also measured. The results of the experiment are outlined in Table 1.

**Table 1. Results of scale-down experiment examining effect of low dissolved oxygen concentrations on the long chain highly unsaturated fatty acid content (DHA % dry weight) of Schizochytrium sp.**

| mL Medium | FAME (%TFA) | DHA (% dry wt) | Biomass (g/L) | Final PH | DO (% sat) |
|---|---|---|---|---|---|
| 50 | 16.5 | 7.4 | 4.2 | 7.4 | 31 |
| 100 | 17.0 | 6.5 | 3.9 | 7.2 | 29 |
| 150 | 22.4 | 9.2 | 2.7 | 7.0 | 11 |
| 200 | 35.9 | 14.5 | 1.8 | 6.9 | 3 |

The results indicate that the lipid content (as % FAME) and DHA content (% dry weight) were higher for cells cultured at low dissolved oxygen levels - the lower the dissolved oxygen level the higher the lipid and DHA content This is unexpected because oxygen had been generally believed to be necessary to form desaturated (double) bonds. It is surprising that so much DHA was formed at low dissolved oxygen level, because DHA is one of the most unsaturated fatty acids. Although the biomass production decreases as the dissolved oxygen level is decreased, the DHA content is increased. Therefore, it is advantageous to have a growth phase with higher dissolved oxygen levels to maximize the formation of biomass and then lower the dissolved oxygen level to maximize long chain fatty acid production.

### Reference Example 3

Microorganisms were produced using fermentors with a nominal working volume of 1,200 gallons. The resulting fermentation broth was concentrated and microorganisms were dried using a drurn-dryer. Lipids from aliquots of the resulting microorganisms were extracted and purified to produce a refined, bleached, and deodorized oil. Approximately 3,000 ppm of d-1-α-tocopheryl acetate was added for nutritional supplementation purposes prior to analysis of the lipid.

Nine fermentations of *Schizochytrium* ATCC No. 20888 were run and the results are shown in Table 2. The dissolved oxygen level was about 8% during the first 24 hours and about 4% thereafter.

**Table 2. Fed-batch fermentation results for the production of DHA from Schizochytrium sp.**

| Entry | Age (Hrs) | Yield¹ (g/L) | DHA² (%) | FAME³ (%) | Productivity⁴ |
|---|---|---|---|---|---|
| 1 | 100.3 | 160.7 | 17.8 | 49.5 | 0.285 |
| 2 | 99.8 | 172.4 | 19.4 | 51.3 | 0.335 |
| 3 | 84.7 | 148.7 | 14.4 | 41.4 | 0.253 |
| 4 | 90.2 | 169.5 | 19.7 | 53.9 | 0.370 |
| 5 | 99.0 | 164.1 | 12.5 | 38.9 | 0.207 |
| 6 | 113.0 | 187.1 | 19.7 | 47.2 | 0.326 |
| 7 | 97.0 | 153.5 | 13.7 | 41.0 | 0.217 |
| 8 | 92.8 | 174.8 | 16.4 | 48.6 | 0.309 |
| Aver.⁵ | 97.1 | 166.4 | 16.7 | 46.5 | 0.288 |
| Std.⁶ | 8.4 | 12.3 | 2.9 | 5.4 | 0.058 |
| CV⁷(%) | 8.7 | 7.4 | 17.3 | 11.7 | 20.2 |

| | | | | | |
|---|---|---|---|---|---|
| 1. actual yield of biomass density. 2. DHA content as % cell dry weight. 3. total fatty acid content as % cell dry weight (measured as methyl esters). 4. (grams of DHA)/L/Hr. 5. average. 6. standard deviation 7. coefficients of variability. Coefficients of variability values below 5% indicate a process which has excellent reproducibility, values between 5% and 10% indicate a process which has good reproducibility and values between 10% and 20% indicate a process which has reasonable reproducibility. | | | | | |

Corn syrup was fed until the volume in the fermentor reached about 1,200 gallons, at which time the corn syrup addition was stopped. The fermentation process was stopped once the residual sugar concentration fell below 5 g/L. The typical age, from inoculation to final, was about 100 hours.

The fermentation broth, i.e., fermentation medium, was diluted with water using approximately a 2: 1 ratio to reduce the ash content of the final product and help improve phase separation during the centrifugation step. The concentrated cell paste was heated to 160° F (about 71° C) and dried on a Blaw Knox double-drum dryer (42"x36"). Preferably, however, microorganisms are dried directly on a drum-dryer without prior centrifugation.

The analysis result of lipids extracted from aliquots of each entries in Table 2 is summarized in Table 3.

**Table 3. Analysis of the microbial biomass produced in the fed-batch fermentations outlined in Table. 2.**

| Entry | % DHA relative to FAME¹ | Total Lipid % by wt. |
|---|---|---|
| 1 | 36.0 | 72.3 |
| 2 | 37.8 | 70.3 |
| 3 | 34.8 | 61.5 |
| 4 | 36.5 | 74.8 |
| 5 | 32.1 | 52.8 |
| 6 | 41.7 | 67.7 |
| 7 | 33.4 | 49.9 |
| 8 | 33.7 | 61.4 |
| Average | 35.8 | 63.8 |
| Std. Deviation³ | 3.0 | 9.1 |
| CV⁴ (%) | 8.5 | 14.2 |

| | | |
|---|---|---|
| 1. see Table 2. 2. see discussion above. 3. standard deviation. 4. coefficients of variability. Coefficients of variability values below 5% indicates a process which has excellent reproducibility, values between 5% and 10% indicates a process which has good reproducibility and values between 10% and 20% indicates a process which has reasonable reproducibility. | | |

Unless otherwise stated, the fermentation medium used throughout the Examples section includes the following ingredients, where the first number indicates nominal target concentration and the number in parenthesis indicates acceptable range: sodium sulfate 12 g/L (11-13); KCl 0.5 g/L (0.45-0.55); MgSO₄•7H₂O 2 g/L (1.8-2.2); Hodag K-60 antifoam 0.35 g/L (0.3-0.4); K₂SO₄ 0.65 g/L (0.60-0.70); KH₂PO₄ 1 g/L(0.9-1.1);(NH₄)₂SO₄ I g/L (0.95-1. 1); CaCl₂•2H₂O 0.17 g/L (0.1 15-0.19); 95 DE corn syrup (solids basis) 4.5 g/L (2-10); MnCl₂•4H₂O 3 mg/L (2.7-3.3); ZnSO₄•7H₂O 3 mg/L (2.7-3.3); COCl₂•6H₂O 0.04 mg/L (0.035-0.045); Na₂MoO₄•2H₂O 0.04 mg/L (0-0.045); CuSO₄•5H₂O 2 mg/L (1.8-2.2); NiSO₄•6H₂O 2 mg/L (1.8-2.2); FeSO₄•7H₂O 10 mg/L (9-11); thiamine 9.5 mg/L (4-15); vitamin B₁₂ 0.15 mg/L (0.05-0.25) and Calcium Pantothenate 3.2 mg/L (1.3-5.1). In addition. 28% NH₄OH solution is used as the nitrogen source.

The ash content of the dried microorganisms is about 6% by weight.

### Example 4

This example illustrates the effect of reduced dissolved oxygen level in the fermentation medium on the productivity of microorganisms at the 14.000-gallon scale.

Using the procedure described in Example 3, a 14,000-gallon nominal volume fermentation was conducted using a wild-type strain *Schizochytrium,* which can be obtained using isolation processes disclosed in the above-mentioned U.S. Patent Nos. 5,340,594 and 5,340,742. The dissolved oxygen level in the fermentation medium was about 8% during the first 24 hours, about 4% from the 24^{th} hour to the 40^{th} hour and about 0.5% from the 40^{th} hour to the end of fermentation process. Results of this lower dissolved oxygen level in fermentation medium processes are shown in Table 4.

**Table 4. Results of 14,000-gallon scale fed-batch fermentations of Schizochytrium at reduced dissolved oxygen concentrations.**

| Entry | Age (Hrs) | Yield (g/L) | %DHA | %FAME | %DHA rel. to FAME | DHA Productivity (g of DHA/L/hr) |
|---|---|---|---|---|---|---|
| 1 | 82.0 | 179.3 | 21.7 | 52.4 | 41.4 | 0.474 |
| 2 | 99.0 | 183.1 | 22.3 | 55.0 | 40.5 | 0.412 |
| 3 | 72.0 | 159.3 | - | - | 40.9 | - |
| 4 | 77.0 | 161.3 | - | - | 43.2 | - |
| 5 | 100.0 | 173.0 | 23.9 | 53.3 | 44.9 | 0.413 |
| 6 | 102.0 | 183.3 | 21.6 | 50.8 | 42.6 | 0.388 |
| 7 | 104.0 | 185.1 | 23.7 | 55.0 | 43.1 | 0.422 |
| 8 | 88.0 | 179.3 | 22.3 | 52.6 | 42.4 | 0.454 |
| 9 | 100.0 | 166.4 | 22.5 | 53.5 | 42.1 | 0.374 |
| 10 | 97.0 | 182.6 | 22.8 | 51.6 | 44.1 | 0.429 |
| 11 | 87.5 | 176.5 | 19.8 | 45.6 | 43.5 | 0.399 |
| 12 | 67.0 | 170.8 | 18.8 | 48.1 | 39.1 | 0.479 |
| 13 | 97.0 | 184.9 | 23.2 | 52.7 | 44.0 | 0.442 |
| 14 | 102.0 | 181.9 | 23.6 | 52.9 | 44.6 | 0.421 |
| 15 | 102.0 | 186.9 | 19.9 | 47.8 | 41.8 | 0.365 |
| 16 | 97.0 | 184.4 | 19.6 | 45.5 | 43.0 | 0.373 |
| 17 | 98.0 | 174.7 | 19.7 | 45.1 | 43.7 | 0.351 |
| 18 | 103.5 | 178.8 | 18.3 | 44.5 | 41.2 | 0.316 |
| 19 | 102.0 | 173.7 | 15.8 | 43.1 | 36.7 | 0.269 |
| 20 | 94.0 | 190.4 | 19.3 | 46.9 | 41.1 | 0.391 |
| 21 | 72.0 | 172.5 | 22.8 | 52.8 | 43.2 | 0.546 |
| 22 | 75.0 | 173.1 | 21.0 | 51.7 | 40.8 | 0.485 |
| 23 | 75.0 | 152.7 | 20.3 | 50.3 | 40.4 | 0.413 |
| 24 | 75.5 | 172.5 | 21.9 | 51.7 | 42.3 | 0.500 |
| 25 | 61.0 | 156.4 | 17.3 | 45.7 | 37.8 | 0.444 |
| 26 | 74.5 | 150.6 | 20.2 | 50.1 | 40.2 | 0.408 |
| 27 | 70.5 | 134.3 | 14.8 | 40.6 | 36.6 | 0.282 |
| 28 | 75.5 | 146.1 | 21.3 | 49.7 | 42.8 | 0.412 |
| 29 | 82.0 | 174.3 | 21.4 | 50.4 | 42.5 | 0.455 |
| 30 | 105.0 | 182.3 | 21.7 | 50.7 | 42.8 | 0.377 |
| 31 | 66.0 | 146.2 | 16.4 | 44.6 | 36.7 | 0.363 |
| | | | | | | |
| Avg | 87.2 | 171.5 | 20.6 | 49.5 | 41.6 | 0.409 |
| Std | 13.9 | 14.1 | 2.4 | 3.8 | 2.3 | 0.061 |
| CV | 16.0% | 8.2% | 11.6% | 7.7% | 5.5% | 15.0% |

### Example 5

This example illustrates the effect of reduced dissolved oxygen level in the fermentation medium on the productivity of microorganisms on a 41,000-gallon scale.

The same procedures as Example 4 were employed except that the fermentation was conducted in a 41,000-gallon fermentor. Culture media volumes were increased to maintain target compound concentrations at this scale. Results are shown in Table 5.

**Table 5. 41,000-gallon scale fermentation of Schizochytrium**

| Entry | Age (Hrs) | Yield (g/L) | %DHA | %FAME | %DHA rel. to FAME | DHA Productivity (g of DHA·L/hr) |
|---|---|---|---|---|---|---|
| 1 | 75.0 | 116.1 | 17.3 | 46.1 | 37.4 | 0.268 |
| 2 | 99.0 | 159.3 | 17.4 | 47.0 | 37.1 | 0.280 |
| 3 | 103.0 | 152.6 | 16.0 | 47.2 | 33.8 | 0.237 |
| 4 | 68.0 | 136.8 | 17.9 | 45.9 | 39.1 | 0.360 |
| 5 | 84.0 | 142.0 | 17.5 | 47.0 | 37.2 | 0.296 |
| Avg | 85.8 | 141.4 | 17.2 | 46.6 | 36.9 | 0.288 |
| Std | 15.1 | 16.6 | 0.7 | 0.6 | 1.9 | 0.046 |
| CV | 17.5% | 11.8 | 4.2% | 1.3% | 5.2% | 15.8% |

### Example 6

This example illustrates the affect of extra nitrogen on the fermentation process of the present invention.

Four sets of 250-L scale fed-batch experiments were conducted using a procedure similar to Example 4. Two control experiments and two experiments containing extra ammonia (1.15x and 1.25x the normal amount) were conducted. Results are shown in Table 6.

**Table 6. Affects of extra ammonia on fermentation of Schizochytrium.**

| Age (hrs) | Yield (g/L) | Biomass Productivity | Conversion Efficiency | DHA Content | FAME Content | DHA Productivity |
|---|---|---|---|---|---|---|
| Sugar target: 7 g/L, Base pH set point: 5.5, Acid pH set point: 7.3, 1.0X NH₃ | | | | | | |
| 48 | 178 | 3.71 g/L/hr | 51.5% | 10.7% | 37.8% | 0.40 g/L/hr |
| 60 | 185 | 3.08 g/L/hr | 46.9% | 16.3% | 47.2% | 0.50 g/L/hr |
| 72 | 205 | 2.85 g/L/hr | 45.2% | 17.4% | 47.4% | 0.50 g/L/hr |
| 84 | 219 | 2.61 g/L/hr | 43.8% | 17.1% | 45.5% | 0.45 g/L/hr |
| 90 | 221 | 2.46 g/L/hr | 44.1% | 18.4% | 48.9% | 0.45 g/L/hr |

| Sugar target: 7 g/L, Base pH set point: 5.5, Acid pH set point: 7.3, 1.15X NH₃ | | | | | | |
|---|---|---|---|---|---|---|
| 48 | 171 | 3.56 g/L/hr | 55.6% | 12.0% | 36.3% | 0.43 g/L/hr |
| 60 | 197 | 3.28 g/L/hr | 54.6% | 9.4% | 38.4% | 0.31 g/L/hr |
| 72 | 191 | 2.65 g/L/hr | 52.8% | 9.4% | 40.0% | 0.25 g/L/hr |
| 84 | 190 | 2.26 g/L/hr | 52.5% | 10.0% | 42.5% | 0.23 g/L/hr |
| 90 | 189 | 2.10 g/L/hr | 52.2% | 9.2% | 43.3% | 0.19 g/L/hr |

| Sugar target: 7 g/L, Base pH set point: 5.5, Acid pH set point: 7.3, 1.25X NH₃ | | | | | | |
|---|---|---|---|---|---|---|
| 48 | 178 | 3.71 g/L/hr | 56.4% | 11.5% | 33.7% | 0.43 g/L/hr |
| 60 | 179 | 2.98 g/L/hr | 48.6% | 10.3% | 36.0% | 0.31 g/L/hr |
| 72 | 180 | 2.50 g/L/hr | 48.8% | 12.0% | 37.6% | 0.30 g/L/hr |
| 84 | 181 | 2.15 g/L/hr | 46.1% | 13.6% | 40.1% | 0.29 g/L/hr |
| 90 | 185 | 2.06 g/L/hr | 45.7% | 12.6% | 40.7% | 0.26 g/L/hr |

| Sugar target: 7 g/L, Base pH set point: 5.5, Acid pH set point: 7.3, 1.0X NH₃ | | | | | | |
|---|---|---|---|---|---|---|
| 48 | 158 | 3.29 g/L/hr | 55.7% | 13.1% | 36.5% | 0.43 g/L/hr |
| 60 | 174 | 2.90 g/L/hr | 48.9% | 17.9% | 39.2% | 0.52g/L/hr |
| 72 | 189 | 2.63 g/L/hr | 45.7% | 21.0% | 39.4% | 0.55 g/L/hr |
| 84 | 196 | 2.33 g/L/hr | 44.1% | 22.4% | 40.1% | 0.52 g/L/hr |
| 90 | 206 | 2.29 g/L/hr | 44.8% | 22.1% | 40.3% | 0.51 g/L/hr |

In general, extra nitrogen has a negative effect on fermentation performance, as significant reductions were observed in the DHA productivity for the two batches where extra ammonia was added. As shown on Table 6, the control batches resulted in final DHA levels of 18.4% and 22.1% of total cellular dry weight versus the 9.2% (1.15x ammonia) and 12.6% (1.25x ammonia) for extra nitrogen supplemented batches.

### Example 7

This example shows a kinetic profile of a fermentation process of the present invention.

A 1000-gallon scale fed-batch experiment was conducted using a procedure similar to Example 4. Kinetic profile of the fermentation process is shown in Table 7.

**Table 7. Kinetic Profile for a 1,000-gallon scale Fed-Batch fermentation of Schizochytrium.**

| Age (hrs) | Yield (g/L) | Biomass Productivity | Conversion Efficiency | %DHA Content | %FAME Content | DHA Productivity |
|---|---|---|---|---|---|---|
| 24 | 118 | 4.92 g/L/hr | 78.2% | 7.4 | 18.8 | 0.36 g/L/hr |
| 30 | 138 | 4.60 g/L/hr | 60.3% | 10.6 | 30.9 | 0.49 g/L/hr |
| 36 | 138 | 3.83 g/L/hr | 46.6% | 11.6 | 36.5 | 0.44 g/L/hr |
| 42 | 175 | 4.17 g/L/hr | 49.8% | 13.4 | 41.7 | 0.56 g/L/hr |
| 48 | 178 | 3.71 g/L/hr | 45.1% | 18.7 | 52.8 | 0.69 g/L/hr |
| 48* | 164 | 3.42 g/L/hr | 41.5% | 15.3 | 33.1 | 0.52 g/L/hr |
| 54 | 196 | 3.63 g/L/hr | 45.7% | 16.6 | 51.2 | 0.60 g/L/hr |
| 60 | 190 | 3.17 g/L/hr | 41.7% | 16.9 | 33.9 | 0.54 g/L/hr |
| 72 | 189 | 2.62 g/L/hr | 39.1% | 15.6 | 31.8 | 0.41 g/L/hr |
| 84 | 195 | 2.32 g/L/hr | 38.5% | 16.4 | 32.7 | 0.38 g/L/hr |
| 90 | 200 | 2.22 g/L/hr | 39.0% | 18.8 | 33.3 | 0.42 g/L/hr |
| 90 | 171 | 1.90 g/L/hr | 33.3% | 22.2 | 61.6 | 0.42 g/L/hr ** |

| | | | | | | |
|---|---|---|---|---|---|---|
| * Two separate samples were analyzed at 48 hrs. ** This is for a washed dry-cell weights (DCW) sample. Other reported values are for unwashed samples. | | | | | | |

### Example 8

This example illustrates affect of the amount of carbon source on productivity.

Three different fermentation processes using the process of Example 4 were conducted using various amounts of carbon source. Results are shown on Table 8.

**Table 8. Fermentation results for various amounts of carbon source on fermentation of Schizochytrium.**

| Age (hrs) | Yield (g/L) | Carbon Charge | Conversion Efficiency | %DHA Content | %FAME Content | Productivity (g/L/hr) |
|---|---|---|---|---|---|---|
| 90 | 171 | 51.3% | 33.3% | 22.2 | 61.6 | 0.42 |
| 94 | 122 | 40.5% | 30.1% | 19.1 | 57.3 | 0.25 |
| 59 | 73 | 20.0% | 36.5% | 11.9 | 40.8 | 0.15 |

### Reference Example 9

This example illustrates the effect of nutrient limitation on carbon conversion efficiency to biomass, lipid and most specifically DHA.

A continuous culture experiment to investigate the effect of nutrient limitation was performed by culturing *Schizochytrium* ATCC No. 20888 in a 2-liter volume Applikon fermentor in basal growth (ICM-2) medium consisting of the following compounds (nominal concentration): Group I ingredients: Na₂SO₄ (18.54 g/L), MgSO₄•7H₂O (2.0 g/L) and KCL (0.572 g/L); Group II ingredients (each prepared separately): glucose (43.81 g/L,), KH₂PO₄ (1.28 g/L), CaCl₂•2H₂O (0.025 g/L) and (NH₄)₂SO₄ (6.538 g/L); Group III ingredients: Na₂EDTA (6.0 mg/L), FeCl₃•6H₂O (0.29 mg/L), H₃BO₃ (6.84 mg/L), MnCl₂•4H₂O (0.86 mg/L), ZnSO₄•7H₂0 (0.237 mg/L), CoCl₂•2H₂O (0.026 mg/L), Na₂MoO₄•2H₂0 (0.005 mg/L), CuSO₄•5H₂0 (0.002 mg/L) and NiSO₄•6H₂0 (0.052 mg/L); and Group IV ingredients: thiamine HCl (0.2 mg/L), Vitamin B₁₂ (0.005 mg/L), Calcium pantothenate (0.2 mg/L). Groups I and II were autoclave sterilized, while Groups III and IV were filter sterilized prior to adding to the fermentor. The growth medium was then inoculated with *Schizochytrium* and grown under controlled conditions of 30°C, pH 5.5 and dissolved oxygen of 20% of saturation until maximum cell density was achieved.

A continuous operation mode was then established by simultaneously pumping sterile ICM-2 feed medium into the fermentor and removing the broth containing *Schizochytrium* cells at a flowrate sufficient to maintain a dilution rate of 0.06hr⁻¹, until a steady state is reached. To investigate the effect of nutrient limitation, the compound containing the specified required nutrient is lowered in the ICM-2 feed medium such that this nutrient is depleted in the outlet cell-containing broth, so that growth of the cells is limited by absence of the particular required nutrient. Once steady state operation was established for each condition, final broth dry biomass, residual glucose, and limiting nutrient concentrations, lipid content of the cell and DHA content of the cells were measured. The conversion efficiency of glucose to biomass was calculated by dividing the total glucose consumed by the total dried biomass formed, and expressed on a percentage basis.

The effects of limiting growth by each individual nutrient were studied by repeating this experiment for each individual nutrient listed in the following table. Final results are summarized in the following table:

**Table 9. Effect of nutrient limitation on the biomass yield. conversion efficiency (glucose -> biomass), lipid content and DHA content of Schizochytrium sp.**

| Limiting Nutrient | Biomass¹ (g/l) | Y_{x/s}² | RCS³ (g/l) | Lipid Content⁴ (%) | DHA Content⁵ (%) |
|---|---|---|---|---|---|
| Glucose | 18.7 | 46.8 | 0.0 | 19.8 | 7.3 |
| Nitrogen | 14.5 | 36.3 | 0.6 | 47.5 | 10.3 |
| Phosphate | 17.8 | 44.5 | 0.8 | 37.0 | 8.2 |
| Thiamine | 7.5 | 18.8 | 7.7 | 11.1 | 4.0 |
| Zinc | 16.0 | 40.0 | 1.3 | 27.8 | 7.2 |
| Copper | 14.0 | 35.0 | 10.4 | 13.8 | 5.3 |
| Cobalt | 14.5 | 36.3 | 0.0 | 22.2 | 6.9 |
| Nickel | 17.8 | 44.5 | 0.0 | 21.9 | 8.0 |
| Iron | 15.9 | 39.8 | 3.5 | 18.5 | 7.2 |
| Manganese | 12.5 | 31.3 | 3.4 | 26.1 | 8.0 |
| Magnesium | 13.9 | 34.8 | 5.3 | 18.7 | 6.4 |
| Calcium | 16.7 | 41.8 | 4.3 | 18.7 | 6.4 |
| Vitamin B12 | 19.6 | 49.0 | 0.0 | 17.5 | 6.3 |
| Molybdenum | 18.9 | 47.3 | 0.0 | 19.3 | 7.0 |
| Pantothenate | 19.2 | 48.0 | 0.0 | 20.4 | 6.7 |
| Sodium | 17.9 | 44.8 | 1.8 | 21.8 | 8.2 |
| Potassium | 13.0 | 32.5 | 8.8 | 14.1 | 5.3 |

| | | | | | |
|---|---|---|---|---|---|
| 1. concentration of dry biomass (grams/liter) 2. yield coefficient (% biomass produced/glucose consumed) 3. residual glucose concentration in broth (grams/liter) 4. lipid content of dry biomass (g lipid (as FAME)/g dry biomass) 5. DHA content of dry biomass (g DHA/g dry biomass) | | | | | |

It is clear from the table that nitrogen limitation resulted in the highest accumulation of DHA in the cells, followed by phosphate, sodium, nickel, manganese, glucose(carbon), zinc and iron. This information can be employed commercially by feeding one or more of these nutrients to a batch fermentation at a rate sufficient to limit cell growth. In the most preferred case, nitrogen is fed in a limiting manner to the batch fermentation to maximize the DHA content of the cells. Other nutrients (or mixtures thereof) can be fed in a limiting manner to maximize production of biomass or other valuable products. Other biologically required elements or nutrients that were not evaluated, such as sulfur, could also be employed as limiting nutrients in this fermentation control strategy.

## Claims

1. A process for producing microbial lipids comprising:
(a) conducting a fermentation of a medium comprising microorganisms, a carbon source and a limiting nutrient source and providing conditions sufficient to maintain a dissolved oxygen level of at least about 4% of saturation in said fermentation medium to increase the biomass density;
(b) subsequently providing conditions sufficient to maintain a dissolved oxygen level of about 1% or less of saturation in said fermentation medium and providing conditions sufficient to allow said microorganisms to produce said lipids; and
(c) recovering said microbial lipids,
wherein at least about 15% of said microbial lipids are polyunsaturated lipids; and
wherein a biomass density of at least 100 g/L is achieved during the fermentation;
wherein said microorganisms are order Thraustochytriales.

2. The process of Claim 1, wherein said fermentation medium or growth medium is at a temperature of at least about 20°C.

3. The process of Claim 1, wherein said process produces lipids at an average rate of at least about 0.5 g/L/hr.

4. The process of Claim 1, wherein said process produces lipids at an average rate of at least about 0.5 *g*/*L*/*hr* and wherein at least about 15% of said lipids are polyunsaturated lipids.

5. The process of Claim 1, wherein said process produces lipids at an average rate of at least about 0.5 g/L/hr and wherein the total amount of omega-3 and omega-6 fatty acids is at least about 20% of said lipids.

6. The process of Claim 1, wherein said process produces lipids at an average rate of at least about 0.5 g/L/hr and wherein at least about 25% of said lipids is docosahexaenoic acid.

7. The process of Claim 1, wherein said microorganisms are selected from the group consisting of *Thraustochytrium, Schizochytrium,* and mixtures thereof.

8. The process of Claim 1, wherein said carbon source is a non-alcohol carbon source.

9. The process of Claim 1, wherein said process produces on average at least about 0.2 g/L/hr of docosahexaenoic acid.

10. The process of Claim 1, wherein said lipid recovery step comprises:
(d) removing water from said fermentation medium to provide dry microorganisms; and
(e) isolating said lipids from said dry microorganisms.

11. The process of Claim 1, wherein said lipid recovery step comprises:
(d) evaporating water from said fermentation medium without prior centrifugation to provide dry microorganisms; and
(e) isolating said lipids from said dry microorganisms.

12. The process of Claim 1, wherein said lipid recovery step comprises:
(d) treating the fermentation broth to permeabilize, lyse or rupture the microbial cells; and
(e) recovering the lipids from the fermentation broth by gravity separation, with or without the aid of an agent to aid in breaking the lipid/water emulsion.

## Patentansprüche

1. Verfahren zur Herstellung von mikrobiellen Lipiden, bei dem man:
(a) eine Fermentation eines Mediums durchführt, das Mikroorganismen, eine Kohlenstoffquelle und eine limitierende Nährstoffquelle umfaßt, und Bedingungen bereitstellt, die geeignet sind, um einen Gehalt an gelöstem Sauerstoff von wenigstens etwa 4% der Sättigung in dem Fermentationsmedium aufrecht zu erhalten, um die Biomassendichte zu erhöhen,
(b) anschließend Bedingungen bereitstellt, die geeignet sind, einen Gehalt an gelöstem Sauerstoff von etwa 1% oder weniger der Sättigung in dem Fermentationsmedium aufrecht zu erhalten, und Bedingungen bereitstellt, die geeignet sind, es den Mikroorganismen zu ermöglichen, die Lipide zu produzieren, und
(c) die mikrobiellen Lipide gewinnt,
wobei wenigstens etwa 15% der mikrobiellen Lipide mehrfach ungesättigte Lipide sind und wobei während der Fermentation eine Biomassendichte von wenigstens 100 g/l erzielt wird,
wobei die Mikroorganismen der Ordnung Thraustochytriales angehören.

2. Verfahren nach Anspruch 1, wobei das Fermentationsmedium oder Wachstumsmedium bei einer Temperatur von wenigstens etwa 20 °C vorliegt.

3. Verfahren nach Anspruch 1, wobei das Verfahren Lipide bei einer durchschnittlichen Geschwindigkeit von wenigstens etwa 0,5 g/l/h hervorbringt.

4. Verfahren nach Anspruch 1, wobei das Verfahren Lipide bei einer durchschnittlichen Geschwindigkeit von wenigstens etwa 0,5 g/l/h hervorbringt und wobei wenigstens etwa 15% der Lipide mehrfach ungesättigte Lipide sind.

5. Verfahren nach Anspruch 1, wobei das Verfahren Lipide bei einer durchschnittlichen Geschwindigkeit von wenigstens etwa 0,5 g/l/h hervorbringt und wobei die Gesamtmenge an Omega-3- und Omega-6-Fettsäuren wenigstens etwa 20% der Lipide ausmacht.

6. Verfahren nach Anspruch 1, wobei das Verfahren Lipide bei einer durchschnittlichen Geschwindigkeit von wenigstens etwa 0,5 g/l/h hervorbringt und wobei wenigstens etwa 25% der Lipide Docosahexaensäure sind.

7. Verfahren nach Anspruch 1, wobei die Mikroorganismen ausgewählt sind aus der Gruppe, bestehend aus *Thraustochytrium, Schizochytrium* und Gemischen davon.

8. Verfahren nach Anspruch 1, wobei die Kohlenstoffquelle eine Kohlenstoffquelle ist, die kein Alkohol ist.

9. Verfahren nach Anspruch 1, wobei das Verfahren durchschnittlich wenigstens etwa 0,2 g/l/h Docosahexaensäure produziert.

10. Verfahren nach Anspruch 1, wobei man bei der Stufe der Lipidgewinnung:
(d) Wasser aus dem Fermentationsmedium entfernt, um trockne Mikroorganismen bereitzustellen, und
(e) die Lipide aus den trocknen Mikroorganismen isoliert.

11. Verfahren nach Anspruch 1, wobei man bei der Stufe der Lipidgewinnung:
(d) Wasser aus dem Fermentationsmedium ohne vorheriges Zentrifugieren evaporiert, um trockene Mikroorganismen bereitzustellen, und
(e) die Lipide aus den trockenen Mikroorganismen isoliert.

12. Verfahren nach Anspruch 1, wobei man bei der Stufe der Lipidgewinnung:
(d) die Fermentationsbrühe behandelt, um die mikrobiellen Zellen permeabel zu machen, sie zu lysieren oder sie zu zerreißen, und
(e) die Lipide aus der Fermentationsbrühe durch Schwerkrafttrennung gewinnt, mit oder ohne der Hilfe eines Mittels, um das Aufbrechen der Lipid- / Wasser-Emulsion zu unterstützen.

## Revendications

1. Procédé pour la production de lipides microbiens, comprenant :
(a) la conduite d'une fermentation d'un milieu comprenant des microorganismes, une source de carbone et une source nutritive limitante et la fourniture de conditions suffisantes pour maintenir un taux d'oxygène dissous d'au moins environ 4 % de la saturation dans ledit milieu de fermentation pour augmenter la biomasse ;
(b) puis la fourniture de conditions suffisantes pour maintenir un taux d'oxygène dissous approximativement égal ou inférieur à 1 % de la saturation dans ledit milieu de fermentation et la fourniture de conditions suffisantes pour permettre auxdits microorganismes de produire lesdits lipides ; et
(c) la récupération desdits lipides microbiens, dans lequel au moins environ 15 % desdits lipides microbiens sont constitués de lipides polyinsaturés ; et
dans lequel une densité de biomasse d'au moins environ 100 g/l est obtenue au cours de la fermentation, lesdits microorganismes appartenant à l'ordre de Thraustochytriales.

2. Procédé suivant la revendication 1, dans lequel ledit milieu de fermentation ou milieu de croissance est à une température d'au moins environ 20°C.

3. Procédé suivant la revendication 1, ledit procédé produisant des lipides à une vitesse moyenne d'au moins environ 0,5 g/l/h.

4. Procédé suivant la revendication 1, ledit procédé produisant des lipides à une vitesse moyenne d'au moins environ 0,5 g/l/h, et dans lequel au moins environ 15 % desdits lipides sont constitués de lipides polyinsaturés.

5. Procédé suivant la revendication 1, ledit procédé produisant des lipides à une vitesse moyenne d'au moins environ 0,5 g/l/h, et dans lequel la quantité totale d'acides gras oméga-3 et oméga-6 est égale à au moins environ 20 % desdits lipides.

6. Procédé suivant la revendication 1, ledit procédé produisant des lipides à une vitesse moyenne d'au moins environ 0,5 g/l/h, et dans lequel au moins environ 25 % desdits lipides sont constitués d'acide docosahexaénoïque.

7. Procédé suivant la revendication 1, dans lequel lesdits microorganismes sont choisis dans le groupe consistant en *Thraustochytrium, Schizochytrium* et leurs mélanges.

8. Procédé suivant la revendication 1, dans lequel ladite source de carbone est une source de carbone sans alcool.

9. Procédé suivant la revendication 1, ledit procédé produisant en moyenne au moins environ 0,2 g/l/h d'acide docosahexaénoïque.

10. Procédé suivant la revendication 1, dans lequel ladite étape de récupération des lipides comprend :
(d) l'élimination de l'eau dudit milieu de fermentation pour fournir des microorganismes secs ; et
(e) l'isolement desdits lipides desdits microorganismes secs.

11. Procédé suivant la revendication 1, dans lequel ladite étape de récupération des lipides comprend :
(d) l'évaporation de l'eau dudit milieu de fermentation sans centrifugation préalable pour fournir des microorganismes secs ; et
(e) l'isolement desdits lipides desdits microorganismes secs.

12. Procédé suivant la revendication 1, dans lequel ladite étape de récupération des lipides comprend :
(d) le traitement du bouillon de fermentation pour perméabiliser, lyser ou rompre les cellules microbiennes ; et
(e) la récupération des lipides à partir du bouillon de fermentation par séparation par gravité, avec ou sans l'assistance d'un agent pour faciliter la rupture de l'émulsion lipide/eau.
